# EUROPEAN PATENT APPLICATION

(11) **EP 4 768 591 A1**
(43) Date of publication of application: **01.07.2026**
(21) Application number: 24916164.7
(22) Date of filing: 23.01.2024
(51) Int. Cl.: C12N 15/81, C12N 1/19, C12N 15/53, C12N 15/54, C12N 15/55, C12N 15/61, C12N 9/90, C12N 9/14, C12N 9/02, C12N 9/10, C12N 9/04, C12P 33/20, C12R 1/865

(54) **METHOD FOR CONSTRUCTING ENGINEERED YEAST FOR DE NOVO SYNTHESIS OF MOGROSIDE**

(30) Priority: 11.01.2024 CN 202410045619
(71) Applicant: Guilin Layn Synthetic Biotechnology Co., Ltd., Guilin, Guangxi 541199 (CN); Jiangnan University, Wuxi, Jiangsu 214122 (CN)
(72) Inventor: LIU, Long, Guilin, Guangxi 541199 (CN); CHEN, Jian, Guilin, Guangxi 541199 (CN); SONG, Yunfei, Guilin, Guangxi 541199 (CN); XIE, Yongfu, Guilin, Guangxi 541199 (CN); LYU, Xueqin, Guilin, Guangxi 541199 (CN); WEI, Fengrui, Guilin, Guangxi 541199 (CN); DU, Guocheng, Guilin, Guangxi 541199 (CN); QU, Guanyi, Guilin, Guangxi 541199 (CN)
(74) Representative: Sun, Yiming
(86) International application number: PCT/CN2024/073548
(87) International publication number: WO 2025/148100

(57) **Abstract**

The present invention discloses a method for constructing an engineered yeast for de novo synthesis of mogrosides. The method includes: inserting a constructed cucurbitadienol synthase gene expression cassette P_{tef1}-*cds*-T_{cyc1}, a constructed epoxide hydrolase gene expression cassette P_{tef1}-*eph3*-T_{cyc1}, a constructed cytochrome P450 enzyme-coded gene CYP and cytochrome P450 enzyme reductase gene expression cassette T_{tdh3}-*cyp87d18*-P_{gal1/10}-*atcpr2*-T_{cyc1}, and a constructed glycosyltransferase-encoded gene expression cassette T*_{adh1}-ugt74ac1-*P_{*gal1*/*10*}-*ugtms1-*T*_{cyc1}* into a yeast genome, to obtain a mogroside-producing engineered yeast. In this way, the efficient fermentation and de novo synthesis of mogrosides is enabled using glucose as a substrate.

## Description

### TECHNICAL FIELD

The present invention relates to a method for constructing an engineered yeast, and in particular to a method for constructing engineered Saccharomyces cerevisiae for de novo synthesis of mogrosides.

### DESCRIPTION OF RELATED ART

Mogrosides are a class of cucurbitane-type tetracyclic triterpenoid secondary metabolites derived from the fruit of Momordica grosvenori, where mogrol serves as an aglycone to link different numbers of glycosyl groups via different glycosidic bonds at C-3 and C-24. They include various structures such as mogroside I A1, mogroside II E, mogroside IlIx, siamenoside I, mogroside IV A, and mogroside V. Among the numerous mogrosides, mogroside V is a component with high content and sweetness and is also the main source of sweetness in the fruit of Momordica grosvenori. Its chemical formula is C₆₀H₁₀₂O₂₉, with the relative molecular weight of 1287.43. In 1995, mogroside V was certified as safe and non-toxic by the FDA, permitting it to be used as a food additive. Due to features such as high sweetness, low calories and non-fermentable nature, mogroside V does not cause obesity or tooth decay, making it an ideal natural sugar substitute. Despite lower sweetness than mogroside V, other mogrosides haven been widely applied to the production of medicaments or cosmetics, because they generally exhibit anti-inflammatory properties and allow for regulating human glucose or lipid metabolism. Hence, mogrosides show broad application prospects.

At present, the industrial production of mogrosides primarily involves extraction from the fruit of Momordica grosvenori. However, the average content of mogrosides in the dried fruit of Momordica grosvenori is less than 1% by weight. Consequently, in case of direct extraction, a great amount of the fruit of Momordica grosvenori is required, the cultivation of which, however, is limited and susceptible to soil and climatic conditions, resulting in low extraction efficiency, increased cost and wastes. At the turn of this century, the rise of synthetic biology broke the limitations of producing specific products from specific species, making it possible to produce mogrosides by microbial synthesis. The microbial synthesis of mogrosides is not constrained by natural conditions, has a shorter production cycle, allows for easy separation of products that can be secreted outside cells and minimal contamination, making it suitable for large-scale production.

Yeasts can be used for brewing and making food such as bread and steamed buns, and can also function as chassis cells for producing natural products along with the development in gene editing technology. Due to advantages such as low pathogenicity, high stress tolerance and reduced susceptibility to phage contamination, yeasts play an important role in the field of genetic engineering. However, the yeasts lack the metabolic pathway for mogroside synthesis. To date, there are not relevant reports on the microbial fermentation method for synthesizing mogrosides from scratch using glucose as a substrate.

### BRIEF SUMMARY OF THE INVENTION

The technical problem to be solved by the present invention is to provide a method for constructing an engineered yeast for de novo synthesis of mogrosides. This method involves integrating exogenous genes into a yeast genome, and regulating and modifying a yeast by means of metabolic engineering, enabling the efficient fermentation and de novo synthesis of mogrosides using glucose as a substrate by means of the yeast.

Specifically, a method for constructing an engineered yeast for de novo synthesis of mogrosides includes: inserting a constructed cucurbitadienol synthase gene expression cassette P*_{tef1}-cds-*T*_{cyc1},* a constructed epoxide hydrolase gene expression cassette P*_{tef1}-eph3-*T*_{cyc1},* a constructed cytochrome P450 enzyme-encoded gene CYP and cytochrome P450 enzyme reductase gene expression cassette T*_{tdh3}-cyp87d18-*P_{*gal1*/}*₁₀-atcpr2-*T*_{cyc1}* and a constructed glycosyltransferase-encoded gene expression cassette T*_{adh1}-ugt74ac1-*P_{*gal1*/}*₁₀-ugtms1-*T*_{cyc1}* into a yeast gene, to obtain a mogroside-producing engineered yeast (hereinafter referred to as recombinant engineered yeast).

Further, a truncated 3-hydroxyl-3-methylglutaryl coenzyme A reductase gene expression cassette P*_{tef1}-thmg1-*T*_{hmg1}* is constructed, and inserted into site *gal80* in the genome of the recombinant engineered yeast. In this way, the rate limiting step of a mevalonate (MVA) pathway is omitted, and meanwhile, the induction by galactose is eliminated, such that the synthesis pathway of mogrosides is inactivated only under the regulation of glucose concentration.

Further, endogenous promoters for a hydroxymethylglutaryl coenzyme A synthase gene *erg13* and a squalene synthetase *erg9* in the recombinant engineered yeast are substituted with strong promoters P*_{tef1},* thereby enhancing the accumulation of mogroside precursors.

Further, a copy number of a squalene epoxidase gene *erg1* of the recombinant engineered yeast is increased, to promote the flow of intermediate squalene toward the target product, mogroside.

Further, a lanosterol synthetase gene *erg7,* a key enzyme in a bypass lanosterol pathway, in the recombinant engineered yeast is knocked out.

Further, in order to enable multi-copy expression of *cyp87d18,* multi-copy plasmids pESC-G418-T*_{tdh3}-cyp87d18-*P_{*gal1*/}*₁₀-atcpr2-*T*_{cyc1}* are transformed into the recombinant engineered yeast, to acquire an optimized engineered yeast.

In the present invention, the engineered yeast includes, but is not limited to, *Saccharomyces cerevisiae, Pichia pastoris, Pichia fermentans, Hansenula anomalies, Yarrowia lipolytica, Candida antarctica, Candida utilis, Candida tropicalis, Schizosaccharomyces pombe,* and *Rhodotorula glutinis.*

In an embodiment, the engineered yeast is *Saccharomyces cerevisiae,* with the cucurbitadienol synthase gene expression cassette P*_{tef1}-cds-*T*_{cyc1}* inserted into site 1622b of the yeast genome; the epoxide hydrolase gene expression cassette P*_{tef1}*-*eph3-*T*_{cyc1}* inserted into site 911b of the yeast genome; the cytochrome P450 enzyme-encoded gene CYP and cytochrome P450 enzyme reductase gene expression cassette T*_{tdh3}-cyp87d18-*P_{*gal1*/}*₁₀-atcpr2-*T*_{cyc1}* inserted into site 106a of the yeast genome; the glycosyltransferase-encoded gene expression cassette T*_{adh1}-ugt74ac1-*P_{*gal1*/}*₁₀-ugtms1-*T*_{cyc1}* inserted into site 308a of the yeast genome; and the truncated 3-hydroxyl-3-methylglutaryl coenzyme A reductase gene expression cassette P*_{tef1}*-*thmg1-*T*_{hmg1}* inserted into site *gal80* of the yeast genome.

The present invention further provides an engineered yeast for de novo synthesis of mogrosides.

Specifically, in the engineered yeast for de novo synthesis of mogrosides, a yeast genome includes a cucurbitadienol synthase gene expression cassette P*_{tef1}-cds-*T*_{cyc1},* an epoxide hydrolase gene expression cassette P*_{tef1}-eph3-*T*_{cyc1},* a cytochrome P450 enzyme-coded gene CYP and cytochrome P450 enzyme reductase gene expression cassette T*_{tdh3}-cyp87d18-*P_{*gal1*/}*₁₀-atcpr2-*T*_{cyc1}* and a glycosyltransferase-encoded gene expression cassette T*_{adh1}-ugt74ac1-*P_{*gal1*/}*₁₀-ugtms1-*T*_{cyc1}.*

The yeast genome includes a truncated 3-hydroxyl-3-methylglutaryl coenzyme A reductase gene expression cassette P*_{tef1}-thmg1-*T*_{hmg1}.*

Endogenous promoters for a hydroxymethylglutaryl coenzyme A synthase gene *erg13* and a squalene synthetase *erg9* in the yeast are strong promoters P*_{tef1}.*

A copy number of squalene epoxidase genes (*erg1*) in the yeast is 3 or more.

A lanosterol synthetase gene *erg7,* a key enzyme in a bypass lanosterol pathway, is knocked out from the yeast.

*Saccharomyces cerevisiae* further includes multi-copy plasmids pESC-G418-T*_{tdh3}-cyp87d18-*P_{*gal1*/}*₁₀-atcpr2-*T*_{cyc1}.*

Further, the yeast is *Saccharomyces cerevisiae, Pichia pastoris, Pichia fermentans, Hansenula anomalies, Yarrowia lipolytica, Candida antarctica, Candida utilis, Candida tropicalis, Schizosaccharomyces pombe,* or *Rhodotorula glutinis.*

In an embodiment, the engineered yeast is *Saccharomyces cerevisiae,* with a genome in which: the cucurbitadienol synthase gene expression cassette P*_{tef1}-cds-*T*_{cyc1}* is inserted into site 1622b; the epoxide hydrolase gene expression cassette P*_{tef1}*-*eph3-*T*_{cyc1}* is inserted into site 911b; the cytochrome P450 enzyme-encoded gene CYP and cytochrome P450 enzyme reductase gene expression cassette T*_{tdh3}-cyp87d18-*P_{*gal1*/}*₁₀-atcpr2-*T*_{cyc1}* is inserted into site 106a; the glycosyltransferase-encoded gene expression cassette T*_{adh1}-ugt74ac1-*P_{*gall1*/}*₁₀-ugtms1-*T*_{cyc1}* is inserted into site 308a; and the truncated 3-hydroxyl-3-methylglutaryl coenzyme A reductase gene expression cassette P*_{tef1}-thmg1-*T*_{hmg1}* is inserted into site *gal80.*

The present invention further provides use of the engineered yeast for de novo synthesis of mogrosides, in particular in biofermentation production of mogrosides.

In the present invention, a method for fermentation production of mogrosides with the engineered yeast includes: placing engineered yeast strains in a liquid YPD medium, culturing the engineered yeast strains for 18 h on a shaker at 30°C and 220 r/min to obtain a fermented seed solution; and transferring the fermented seed solution into a fermentation medium at a inoculation rate of 3%, and culturing the fermented seed solution for 96 h at 30°C and 220 r/min.

Beneficial effects of the present invention:
1. The *Saccharomyces cerevisiae* provided by the present invention enable fermentation production of mogrosides with glucose as a substrate, and products can be secreted to outside of cells, laying a solid foundation for downstream separation and purification steps. The *Saccharomyces cerevisiae* has an endogenous MVA pathway, allowing for synthesis of squalene (a key precursor for mogrosides), which is then catalyzed by exogenous squalene epoxidase, cucurbitadienol synthase, epoxide hydrolase, a cytochrome P450 enzyme (and its concomitant protein, cytochrome P450 reductase), and glycosyltransferase in sequence to synthesize mogrosides.
   The yield of mogroside V produced by a final *Saccharomyces cerevisiae* strain Mogrol 09 provided by the present invention reaches 5.6 mg/L, which is 467 times higher than that of an initial *Saccharomyces cerevisiae* strain Mogrol 04 that allows for synthesis of mogroside V. The yields of other mogrosides such as mogroside IIE, mogroside Illx, mogroside IVA and siamenoside produced by the final strain Mogrol 09 are also significantly higher than those of the strain Mogrol 04 that allows for preliminary synthesis of mogroside V.
2. The yeast provided by the present invention is simple and is convenient to use, showing promising prospects.

In the present invention, key enzymes, such as a cucurbitadienol synthase CDS, an epoxide hydrolase EPH, a cytochrome P450 enzyme, a cytochrome P450 enzyme reductase, and glycosyltransferases UGT74AC1 and UGTMS1, for the synthesis pathway (i.e., the metabolic pathway for mogroside synthesis) are integrated into the yeast using the CRISPR Cas9 gene editing technology, enabling fermentation and de novo synthesis of mogrosides by means of the yeast using glucose as the substrate.

4. With the yeast provided by the present invention, the promoters P*_{tef1}* and P_{*gal1*/*10*} are used to control the expression of pathway genes for the mogroside synthesis; the truncated 3-hydroxyl-3-methylglutaryl coenzyme A reductase tHMG1 is integrated; the copy number of squalene epoxidase genes *erg1* is increased; the key lanosterol synthetase gene *erg7,* a key enzyme in the bypass lanosterol pathway, is knocked out, to avoid competition for mogroside precursors in the bypass pathway; and the multi-copy plasmids pESC-G418*-*T*_{tdh3}-cyp87d18-*P_{*gal1*/}*₁₀-atcpr2-*T*_{cyc1}* are transformed to enable multi-copy expression of cyp87d18. In this way, the yeast allows for efficient fermentation and de novo synthesis of mogroside using the glucose as the substrate.

### BRIEF DESCRIPTION OF THE SEVERAL VIEWS OF THE DRAWINGS

FIG. 1 shows a comparison of yields of Saccharomyces cerevisiae strains Mogrol 09 and Mogrol 04; and
FIG. 2 shows the yields of other mogrosides produced by Saccharomyces cerevisiae strains Mogrol 09 and Mogrol 04.

### DETAILED DESCRIPTION OF THE INVENTION

The specific embodiments of the present invention will be described in detail below. These embodiments are provided to enable a more thorough understanding of the present invention and to fully convey the scope of the present invention to those skilled in the art.

A sample treatment method in the examples of the present invention is as follows:
a fermentation broth is collected and centrifuged for 5 min at 8,000 r/min, a chromatographic-grade formic acid was added to 1 mL of a resulting supernatant till the final concentration of the formic acid reaches 3% (v/v); and at 4°C, a resulting mixture was let stand for 4 h to 8 h to remove proteins, and then centrifuged for 5 min at 8,000 r/min, 0.5 mL of a resulting supernatant was filtered through a 0.22 µm aqueous filter membrane and then added to a liquid chromatography testing flask for testing of yield.

A testing method in the examples of the present invention is as follows:
testing method of high-performance liquid chromatography-mass spectrometry (LC-MS) instrument: Waters (MICROMASS QUATTRO MICRO); BEH-C18 column; mobile phase: acetonitrile and 0.1% aqueous formic acid solution; column temperature: 45°C; injection volume: 1 µL.

### Example 1: Construction of Mogroside-producing Strains

According to the amino acid sequences of key enzymes for mogroside synthesis as published on the National Center of Biotechnology Information (NCBI) or sourced from references, the following amino acid sequences of key enzymes were included: cucurbitadienol synthase CDS (NCBI ID: AUC64917.1), with an amino acid sequence as set forth in SEQ ID NO. 1; epoxide hydrolase EPH, with an amino acid sequence as set forth in SEQ ID NO. 2; cytochrome P450 enzyme CYP87D18 (NCBI ID: K7NBR2.1), with an amino acid sequence as set forth in SEQ ID NO. 3; cytochrome P450 enzyme reductase AtCPR2 (NCBI ID: UTD45115.1), with an amino acid sequence as set forth in AA SEQ ID NO. 4; glycosyltransferase UGT74AC1, with an amino acid sequence as set forth in SEQ ID NO. 5; and glycosyltransferase UGTMS1, with an amino acid sequence as set forth in SEQ ID NO.6.

A truncated 3-hydroxyl-3-methylglutaryl-coenzyme A reductase tHMG1 was obtained by removing an N-terminus transmembrane domain consisting of amino acids 2 to 530 from an endogenous Saccharomyces cerevisiae enzyme HMG1, with an amino acid sequence as set forth in SEQ ID NO. 7.

Codon optimization and total gene synthesis were conducted according to the codon preference of Saccharomyces cerevisiae.

Based on the gene sequences at integration sites 1622b, 911b, 106a, and 308a in the Saccharomyces cerevisiae genome, a 20 bp PAM sequence of CRISPR Cas9 was selected; primers for amplifying the upstream and downstream homologous arms of the gene expression cassette were designed flanking the PAM sequence; and the upstream and downstream homologous arms were amplified by PCR using the DNA of Saccharomyces cerevisiae genome as a template.

Primer conditions are shown in Table 1. PCR conditions are shown in Table 2.

**Table 1. Primers required for homologous arm amplification**

| Primer name | Primer sequence |
|---|---|
| 1622bU-F | GAGCCGCATCAATGCTATCGA |
| 1622bU-R | CGAAGGCTTTAATTTGCGGCCCGAACTTTACGAATCTTGACG ACTACATGA |
| 1622bD-F | CTCAGGTATAGCATGAGGTCGCTCACACTCCCGACATTACGC T |
| 1622bD-R | CCAGCACAATACCATATACCAACG |
| 911bU-F | CTTCTACCGCAACAGGTTGCTTAAATTTATTTAGATG |
| 911bU-R | GACGCTCGAAGGCTTTAATTTGCAAGCGCCCATTCAACATCC GATTTTTTTCTATAAAATAAG |
| 911bD-F | GGAGTAGAAACATTTTGAAGCTATGAAATAGCATACAAAACAG ACATAAAATTTAAAACAGAAATG |
| 911bD-R | GTCAAGTATCAACCTCGATAAATTAGAATCTTTGCCAG |
| 106a-U-F | GATATTCCTAAGCCTCCCTCACCA |
| 106a-U-R | GACGCTCGAAGGCTTTAATTTGCGGCCCACAACCGACGATC CGGG |
| 106a-D-F | CGCTGTATAGCTCATATCTTTCCCTTCGCTACCCGAAAGTTTT TCCG |
| 106a-D-R | CTGGCGTTGTCAATGACAGACTT |
| 308a-U-F | GCTCCCCTGGATTTTATGCGAAG |
| 308a-U-R | GAAGGTTTTGGGACGCTCGAAGGCTTTAATTTGCGGCCCGA AGTTAATGTTGAAATTTCACTTATTTTAGTCATCTTTG |
| 308a-D-F | GTCAGGTTGCTTTCTCAGGTATAGCATGAGGTCGCTCCTACA TATTGCTACCACTTCTATTACACAATAGTTTCAAT |
| 308a-D-R | GTAAACCCATCAACATATAGCTTAGATGTTCAAAAAC |

**Table 2. PCR amplification conditions**

| Step | Temperature (°C) | Time |
|---|---|---|
| 1. Pre-denaturation | 98 | 3 min |
| 2. Denaturation | 98 | 10 s |
| 3. Annealing | 55 | 15 s |
| 4. Extension | 72 | 6 kb/min |
| 5. Extension | 72 | 5 min |
| Steps 2 to 4 were perform ed for 30 cycles. | | |

Primers were designed following an overlap extension PCR method to amplify key enzymes in the mogroside synthesis pathway, as well as the promoters and terminators involved. Plasmids *cds, eph3, cyp87d18, atcpr2, ugtac1,* and *ugtms1* in full-gene synthesis were used as templates for amplification to obtain corresponding gene fragments. The *Saccharomyces cerevisiae* genome DNA was used as a template for amplification to obtain promoters P*_{tef1}* and P_{*gal1*/*10*} and terminators T*_{cyc1},* T*_{adh1}* and T*_{tdh3}.* Gene expression cassettes were constructed by the overlap extension PCR.

Primer conditions are shown in Table 3. PCR amplification conditions are shown in Table 2.

**Table 3. Primers required for fragment amplification**

| Primer name | Primer sequence |
|---|---|
| TEF1(CD S)-F | ATAGCTTCAAAATGTTTCTACTCCTT |
| TEF1 (CD S)-R | AGCACCAACTTTCAATCTCCACATAAACTTAGATTAGATTGCTATGC TTTCT |
| TEF1 (EP H)-F | ATAGCTTCAAAATGTTTCTACTCCTT |
| TEF1 (EP H)-R | GTGTTCAATTTGGTCCATAAACTTAGATTAGATTGCTATGCTTTCTT TCTAATGAGCA |
| GAL1/10( CYP)-F | GAAGAAGAGGACATTTATATTGAATTTTCAAAAATTCTTACTTTTTTT TTGGATGGACG |
| GAL1/10( CYP)-R | CAACAGTCCACATTATAGTTTTTTCTCCTTGACGTTAAAGTATAGAG GTATATTAAC |
| GAL1/10( UGT)-F | GTTGTAGTATCACCTTGTTGAGCAGCATCCATGTTTTTTCTCCTTG ACGTTAAAGTATAGAGGTATATTAACAATTTTTTG |
| GAL1/10( UGT)-R | CCAAAATATGAGTATCACCTTTTTCCATTTTCAAAAATTCTTACTTTT TTTTTGGATGGACGCAAAG |
| CDS-F | AAAGCATAGCAATCTAATCTAAGTTTATGTGGAGATTGAAAGTTGGT GCT |
| CDS-R | AATAAAAATCATAAATCATAAGAAATTCGCTTATTCAGTCAAAACTCT ATGACAATATTCAC |
| EPH3-F | GTAAGCGTGACATAACTAATTACATGACTAGAGCTCGAACTTGTTG ATGAAATCATGG |
| EPH3-R | GTAAGCGTGACATAACTAATTACATGACTAGAGCTCGAACTTGTTG ATGAAATCATGG |
| CYP-F | CTCTATACTTTAACGTCAAGGAGAAAAAACTATAATGTGGACTGTTG TTTTGGGTTTGG |
| CYP-R | GATTTAAAGTAAATTCACTTTATTCTTTTGGAGTAAATTTAACATGCA AACCATCTTC |
| CPR-F | GCGTGACATAACTAATTACATGATTACCATACATCTCTAAGATATCTT CCACTCGTTTG |
| CPR-R | GAATTTTTGAAAATTCAATATAAATGTCCTCTTCTTCTTCTTCGTCAA CCTC |
| AC1-F | CTTCTTTGCGTCCATCCAAAAAAAAAGTAAGAATTTTTGAAAATGG AAAAAGGTGATACTCATATTTTGGTTTTTCC |
| AC1-R | CTTATTTAATAATAAAAATCATAAATCATAAGAAATTCGCTTAAGTTTG |
| | TTTCAACATAGCAACAAATTCTTCAATATTTTTATCAG |
| MS1-F | GTAAGCGTGACATAACTAATTACATGATTAGATTTTCAATAACAAAG AAATTTCAGCAACCATTTCATC |
| MS1-R | CCTCTATACTTTAACGTCAAGGAGAAAAAACATGGATGCTGCTCAA CAAGGTGATAC |
| CYC1(MS 1)-F | GGCCGCAAATTAAAGCCTTCGAG |
| CYC1(MS 1)-R | GCTGAAATTTCTTTGTTATTGAAAATCTAATCATGTAATTAGTTATGT CACGCTTACATTCACG |
| CYC1(CP R)-F | GGCCGCAAATTAAAGCCTTCGAG |
| CYC1(CP R)-R | CTTAGAGATGTATGGTAATCATGTAATTAGTTATGTCACGCTTACATT CACG |
| CYC1 (EP H)-F | GGCCGCAAATTAAAGCCTTCG |
| CYC1 (EP H)-R | ATTTCATCAACAAGTTCGAGCTCTAGTCATGTAATTAGTTATGTCAC GCTTACATTCACG |
| ADH1(UG T)-F | TTGTTGCTATGTTGAAACAAACTTAAGCGAATTTCTTATGATTTATG ATTTTTATTATTAAATAAGTTATAAAAAAAATAAGTGT |
| ADH1(UG T)-R | CAGGTATAGCATGAGGTCGCTC |
| ADH1(CD S)-F | ATAGAGTTTTGACTGAATAAGCGAATTTCTTATGATTTATGATTTTTA TTATTAAATAAG |
| ADH1(CD S)-R | GAGCGACCTCATGCTATACCTGAG |
| TDH3-F | CTCCAAAAGAATAAAGTGAATTTACTTTAAATCTTGCATTTAAATAAA TTTTCTTTTTATAG |
| TDH3-R | AAGGGAAAGATATGAGCTATACAGCG |

Gene expression cassettes included: a cucurbitadienol synthase gene expression cassette P*_{tef1}-cds-*T*_{cyc1},* a epoxide hydrolase gene expression cassette P*_{tef1}-eph3-*T*_{cyc1},* a cytochrome P450 enzyme-coded gene CYP and cytochrome P450 enzyme reductase gene expression cassette T*_{tdh3}-cyp87d18-*P_{*gal1*/}*₁₀-atcpr2-*T*_{cyc1},* and a glycosyltransferase-encoded gene expression cassette T*_{adh1}-ugt74ac1-*P_{*gal1*/*10*}-*ugtms1-*T*_{cyc1}.*

The promoter P*_{tef1}* was a constitutive promoter endogenous to *Saccharomyces cerevisiae,* with a nucleotide sequence as set forth in NA SEQ ID NO. 8. The promoter P_{*gal1*/*10*} was a bidirectional promoter composed of promoters *gal1* and *gal10,* with a nucleotide sequence as set forth in NA SEQ ID NO. 9. The terminators T*_{cyc1},* T*_{adh1}* and T*_{tdh3}* were terminators endogenous to *Saccharomyces cerevisiae,* with nucleotide sequences as set forth in NA SEQ ID NO.10, NA SEQ ID NO.11, and NA SEQ ID NO.12, respectively.

A sgRNA plasmid of CRISPR Cas9 was constructed using a plasmid pML104 as a template; circular PCR was performed using primers (as set forth in NA SEQ ID NO. 13) in which the 20 bp PAM sequence was added; and sequencing was performed to obtain a plasmid pML104-PAM with the corresponding PAM sequence.

The constructed pML104-PAM and the gene expression cassettes were transformed into competent cells of host strains of *Saccharomyces cerevisiae* CEN PK2-1C *(MATa; ura3-52; trp1-289; leu2-3,112*; *his3Δ 1; MAL2-8C; SUC2*); transformants with correct PCR results were selected from colonies and sequenced for validation, to ultimately obtain a recombinant *Saccharomyces cerevisiae* strain Mogrol 04 with the mogroside synthesis pathway.

### Example 2: Enhancement of Accumulation of Key Intermediate Products

Mogroside synthesis pertains to secondary metabolism with limited precursor accumulation. In order to improve precursor synthesis and eliminate the limitation by the rate-limiting enzyme hydroxyymethylglutaryl coenzyme A reductase in the mevalonate pathway, an N-terminus endoplasmic reticulum localization signal peptide of a gene *hmg1* was excised, to obtain *thmg1.* A truncated 3-hydroxyl-3-methylglutaryl coenzyme A reductase gene expression cassette P*_{tef1}-thmg1-*T*_{hmg1}* was constructed, transformed into a strain Mogrol 04, and inserted into site *gal80* in a *Saccharomyces cerevisiae* genome, i.e. adding one copy of *thmg1* to site *gal80,* thereby obtaining a strain named Mogrol 05. The strain Mogrol 05 eliminated the rate-limiting step in the MVA pathway, and was not inducible by galactose, such that the synthesis pathway for mogrosides is activated only under the regulation of glucose concentration.

On this basis, key enzymes in the mevalonate pathway were overexpressed; the endogenous promoters for an endogenous hydroxymethylglutaryl coenzyme A synthase gene *erg13* and a squalene synthase *erg9* in the *Saccharomyces cerevisiae* were substituted with strong promoters P*_{tef1},* to obtain a strain Mogrol 06.

### Example 3: Enhancement of Mogroside Synthesis Pathway

In order to promote the flow of more intermediate squalene toward mogrosides, the strain Mogrol 06 was used as a starting strain, the copy number of the squalene epoxidase gene *erg1* was increased to 3, to obtain a strain Mogrol 07. In order to prevent the squalene from flowing to a bypass pathway and competing with the mogroside synthesis pathway for precursors, Mogrol 07 was used as a starting strain, and a lanosterol synthase (a key enzyme) gene *erg7* in a bypass lanosterol pathway was knocked out, to obtain a strain Mogrol 08. Due to low expression level of cytochrome P450 and in order to improve the expression level of *cyp87d18,* the strain Mogrol 08 was used as a starting strain, and multi-copy plasmids pESC-G418-T*_{tdh3}*-*cyp87d18-*P_{*gal1*/}*₁₀-atcpr2-*T*_{cyc1}* were transformed into Mogrol 08, to obtain a strain Mogrol 09.

### Example 4: Fermentation Production of Mogrosides by Recombinant Saccharomyces cerevisiae

Recombinant Saccharomyces cerevisiae strains having the mogroside synthesis pathway were streaked onto SD plates (supplemented with 50 mg/L uracil) and cultured at 30°C till the growth of numerous colonies.

A single colony was inoculated onto a seed medium and cultured for 18 h to 20 h at 30°C and 220 r/min until the early logarithmic phase of cell growth.

A seed culture solution was inoculated into a fermentation medium at an initial inoculum of 3%, and cultured for 96 h at 30°C and 220 r/min. Samples of the bacterial solution were taken every 12 hours to measure OD600 and residual glucose. After 96 h, the culture was stopped; a bacterial solution was transferred into a 50 mL centrifuge tube and centrifuged for 5 min at 8,000 r/min; and a chromatographic-grade formic acid was added to 1 mL of a resulting supernatant till the final concentration of the formic acid reaches 3% (v/v). At 4°C, a resulting mixture was let stand for 4 h to 8 h to remove proteins, and then centrifuged for 5 min at 8,000 r/min; 0.5 mL of a resulting supernatant was filtered through an aqueous filter membrane and then added to a liquid chromatography testing flask for testing of yield.

The seed medium was a liquid YPD medium. The medium ingredients were: 20 g/L glucose, 20 g/L peptone, and 10 g/L yeast powder.

The fermentation medium was a liquid YPD medium with a regulated glucose concentration. The medium ingredients were: 30 g/L glucose, 20 g/L peptone, and 10 g/L yeast powder.

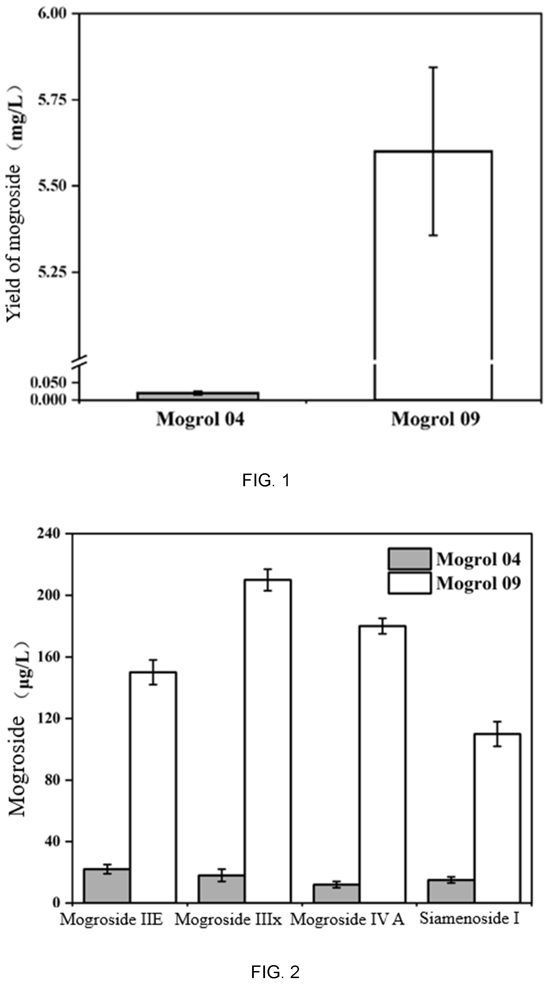

The yields of mogroside V and other mogrosides are shown in FIG. 1 and FIG. 2, respectively. The yield of mogroside V produced by the recombinant strain Mogrol 09 is 5.6 mg/L, which is 467 times higher than that of the strain Mogrol 04 that is initially constructed for synthesis of mogroside V. The yields of other mogrosides provided by the Saccharomyces cerevisiae strain Mogrol 09 are also significantly higher than those by Mogrol 04, by which the yields of mogroside IIE, mogroside IlIx, mogroside IVA, and siamenoside I can reach 150.2 µg/L, 212.3 µg/L, 183.5 µg/L, and 110.4 µg/L, respectively.

## Claims

1. A method for constructing an engineered yeast for de novo synthesis of mogrosides, comprising: inserting a constructed cucurbitadienol synthase gene expression cassette P*_{tef1}-cds-*T*_{cyc1},* a constructed epoxide hydrolase gene expression cassette P*_{tef1}-eph3-*T*_{cyc1},* a constructed cytochrome P450 enzyme-encoded gene CYP and cytochrome P450 enzyme reductase gene expression cassette T*_{tdh3}-cyp87d18-*P_{*gal1*/}*₁₀₋atcpr2-*T*_{cyc1}* and a constructed glycosyltransferase-encoded gene expression cassette T*_{adh1}-ugt74ac1-*P_{*gal1*/}*₁₀-ugtms1-*T*_{cyc1}* into a yeast gene, to obtain a mogroside-producing engineered yeast.

2. The method for constructing the engineered yeast for de novo synthesis of mogrosides according to claim 1, wherein a truncated 3-hydroxyl-3-methylglutaryl coenzyme A reductase gene expression cassette P*_{tef1}-thmg1-*T*_{hmg1}* is constructed, and inserted to site *gal80* in the genome of the mogroside-producing engineered yeast.

3. The method for constructing the engineered yeast for de novo synthesis of mogrosides according to claim 2, wherein endogenous promoters for a hydroxymethylglutaryl coenzyme A synthase gene *erg13* and a squalene synthetase *erg9* in the mogroside-producing engineered yeast are substituted with strong promoters P*_{tef1}.*

4. The method for constructing the engineered yeast for de novo synthesis of mogrosides according to claim 3, wherein a copy number of a squalene epoxidase gene *erg1* of the mogroside-producing engineered yeast is increased, the copy number being 3 or more.

5. The method for constructing the engineered yeast for de novo synthesis of mogrosides according to claim 4, wherein a lanosterol synthetase gene *erg7,* a key enzyme in a bypass lanosterol pathway, in the mogroside-producing engineered yeast is knocked out.

6. The method for constructing the engineered yeast for de novo synthesis of mogrosides according to claim 5, wherein multi-copy plasmids pESC-G418-T*_{tdh3}-cyp87d18-*P_{*gal1*/}*₁₀-atcpr2-*T*_{cyc1}* are transformed into the mogroside-producing engineered yeast.

7. The method for constructing the engineered yeast for de novo synthesis of mogrosides according to any one of claims 1 to 6, wherein the engineered yeast comprises, but is not limited to, *Saccharomyces cerevisiae, Pichia pastoris, Pichia fermentans, Hansenula anomalies, Yarrowia lipolytica, Candida antarctica, Candida utilis, Candida tropicalis, Schizosaccharomyces pombe,* and *Rhodotorula glutinis.*

8. The method for constructing the engineered yeast for de novo synthesis of mogrosides according to any one of claims 2 to 6, wherein the engineered yeast is *Saccharomyces cerevisiae,* with the cucurbitadienol synthase gene expression cassette P*_{tef1}-cds-*T*_{cyc}1* inserted into site 1622b of the yeast genome; the epoxide hydrolase gene expression cassette P*_{tef1}-eph3-*T*_{cyc1}* inserted into site 911b of the yeast genome; the cytochrome P450 enzyme-encoded gene CYP and cytochrome P450 enzyme reductase gene expression cassette T*_{tdh3}-cyp87d18-*P_{*gal1*/}*₁₀-atcpr2-*T*_{cyc1}* inserted into site 106a of the yeast genome; the glycosyltransferase-encoded gene expression cassette T*_{adh1}-ugt74ac1-*P_{*gal1*/}*₁₀-ugtms1-*T*_{cyc1}* inserted into site 308a of the yeast genome; and the truncated 3-hydroxyl-3-methylglutaryl coenzyme A reductase gene expression cassette P*_{tef1}-thmg1-*T*_{hmg1}* inserted into site *gal80* of the yeast genome.

9. An engineered yeast for de novo synthesis of mogrosides, wherein a yeast genome comprises a cucurbitadienol synthase gene expression cassette P*_{tef1}-cds-*T*_{cyc1},* an epoxide hydrolase gene expression cassette P*_{tef1}-eph3-*T*_{cyc1},* a cytochrome P450 enzyme-coded gene CYP and cytochrome P450 enzyme reductase gene expression cassette T*_{tdh3}-cyp87d18-*P_{*gal1*/}*₁₀-atcpr2-*T*_{cyc1}* and a glycosyltransferase-encoded gene expression cassette T*_{adh1}-ugt74ac1-*P_{*gal1*/}*₁₀-ugtms1-*T*_{cyc1}.*

10. The engineered yeast for de novo synthesis of mogrosides according to claim 9, wherein the yeast genome comprises a truncated 3-hydroxyl-3-methylglutaryl coenzyme A reductase gene expression cassette P*_{tef1}-thmg1-*T*_{hmg1}.*

11. The engineered yeast for de novo synthesis of mogrosides according to claim 10, wherein endogenous promoters for a hydroxymethylglutaryl coenzyme A synthase gene *erg13* and a squalene synthetase *erg9* in the yeast are strong promoters P*_{tef1}.*

12. The engineered yeast for de novo synthesis of mogrosides according to claim 11, wherein a copy number of squalene epoxidase genes (*erg1*) in the yeast is 3 or more.

13. The engineered yeast for de novo synthesis of mogrosides according to claim 12, wherein a lanosterol synthetase gene *erg7,* a key enzyme in a bypass lanosterol pathway, is knocked out from the yeast.

14. The engineered yeast for de novo synthesis of mogrosides according to claim 13, wherein *Saccharomyces cerevisiae* further comprises multi-copy plasmids pESC-G418-T*_{tdh3}-cyp87d18-*P_{*gal1*/}*₁₀-atcpr2-*T*_{cyc1}.*

15. The engineered yeast for de novo synthesis of mogrosides according to any one of claims 9 to 14, wherein the engineered yeast comprises, but is not limited to, *Saccharomyces cerevisiae, Pichia pastoris, Pichia fermentans, Hansenula anomalies, Yarrowia lipolytica, Candida antarctica, Candida utilis, Candida tropicalis, Schizosaccharomyces pombe,* and *Rhodotorula glutinis.*

16. The engineered yeast for de novo synthesis of mogrosides according to any one of claims 10 to 14, wherein the engineered yeast is *Saccharomyces cerevisiae,* with a genome in which: the cucurbitadienol synthase gene expression cassette P*_{tef1}-cds-*T*_{cyc1}* is inserted into site 1622b; the epoxide hydrolase gene expression cassette P*_{tef1}-eph3-*T*_{cyc1}* is inserted into site 911b; the cytochrome P450 enzyme-encoded gene CYP and cytochrome P450 enzyme reductase gene expression cassette T*_{tdh3}-cyp87d18-*P_{*gal1*/}*₁₀-atcpr2-*T*_{cyc1}* is inserted into site 106a; the glycosyltransferase-encoded gene expression cassette T*_{adh1}-ugt74ac1-*P_{*gal1*/}*₁₀-ugtms1-*T*_{cyc1}* is inserted into site 308a; and the truncated 3-hydroxyl-3-methylglutaryl coenzyme A reductase gene expression cassette P*_{tef1}-thmg1-*T*_{hmg1}* is inserted into site *gal80.*

17. Use of the engineered yeast for de novo synthesis of mogrosides according to any one of claims 9 to 16 in biofermentation production of mogrosides.

18. A method for fermentation production of mogrosides with the engineered yeast for de novo synthesis of mogrosides according to claim 14, comprising: placing engineered yeast strains in a liquid YPD medium, culturing the engineered yeast strains for 18 h on a shaker at 30°C and 220 r/min to obtain a fermented seed solution; and transferring the fermented seed solution into a fermentation medium at a inoculation rate of 3%, and culturing the fermented seed solution for 96 h at 30°C and 220 r/min.
